# DEMANDE DE BREVET EUROPEEN

(11) **EP 3 375 460 A1**
(43) Date de publication de la demande: **19.09.2018**
(21) Numéro de dépôt: 18161557.6
(22) Date de dépôt: 13.03.2018
(51) Int. Cl.: A61L 27/18, A61L 27/20, A61L 27/54, A61L 31/06, A61L 31/14, A61L 31/16, A61F 2/00, A61K 35/644

(54) **PROTHESE IMPLANTABLE, NOTAMMENT POUR LA CHIRURGIE PARIETALE**

(30) Priorité: 14.03.2017 FR 1752072
(71) Demandeur: Cousin Biotech, 59117 Wervicq Sud (FR)
(72) Inventeur: CATTEAU, Franck, 59117 WERVICQ SUD (FR); SOLECKI, Gilles, 59390 LANNOY (FR)
(74) Mandataire: Cabinet Beau de Loménie

(57) **Abrégé**

La présente invention a pour objet une prothèse implantable (1), notamment pour la chirurgie pariétale, comprenant une pièce textile poreuse (3) comprenant des fils monofilamentaires et ayant au moins une première face (3a), et au moins un revêtement fonctionnel (26), continu ou discontinu, disposé selon tout ou partie de ladite première face, ledit revêtement est dans une composition comprenant un miel ou un mélange de plusieurs miels (25) représentant au moins 50% en masse de la masse totale de ladite composition, et en ce que la masse surfacique de la pièce textile (3) sans revêtement (26) est inférieure ou égale à 150 g/m².

## Description

La présente invention est dans le domaine technique des prothèses implantables, notamment pour la chirurgie pariétale, en particulier pour la réfection des hernies, comprenant une pièce textile et un revêtement disposé selon tout ou partie de l'une des faces de la pièce textile, ledit revêtement étant dans une composition comprenant du miel ou un mélange de plusieurs miels. La prothèse implantable peut ainsi être placée en première intention en cure de la hernie, ou en seconde intention contre une prothèse de réfection pariétale déjà implantée lorsqu'un risque infectieux a été détecté en relation avec cette prothèse. La présente invention a également pour objet un procédé de fabrication d'une telle prothèse implantable.

La survenue d'une infection est un risque pour toute intervention chirurgicale. On retrouve ainsi des bactéries pathogènes dans plus de 90% des plaies opératoires lors de la fermeture de ces dernières. Ce phénomène existe quelle que soit la technique chirurgicale et quel que soit l'environnement (le flux laminaire ne supprime pas complètement ce risque). Ces bactéries sont peu nombreuses mais peuvent proliférer. Elles trouvent dans la plaie opératoire un milieu favorable (hématome, ischémie, modification du potentiel d'oxydoréduction...) et l'intervention induit des anomalies des défenses immunitaires. La chirurgie pariétale ne saurait donc pas échapper à ce risque majeur redouté lors de l'insertion d'une prothèse. Ce risque est variable selon les séries, le site d'implantation et le type de prothèse mise en place. Le nombre de prothèses disponibles sur le marché est important, avec même pour certains matériaux considérés comme tolérants une possibilité de pose en milieu septique. De fait devant cette offre pléthorique et le risque infectieux toujours présent, l'homme du métier ne sait quelle prothèse choisir en particulier en regard du risque infectieux qu'elle peut présenter.

Environ 300 000 (dont environ 200 000 cures de hernie de l'aine) traitements de défects pariétaux sont réalisés chaque année en France par mise en place de matériel prothétique. Le taux d'infection des prothèses pour les cures de hernies de l'aine est estimé à 1,4% en cas d'antibioprophylaxie et à 2,9% en l'absence d'antibioprophylaxie (réfs : la méta analyse de Sanchez-Manuel et al Sanchez-Manuel FJ, Seco-Gil J, Antibiotic prophylaxis for hernia repair, Cochrane Database Syst Rev 2009).

Pour la chirurgie des éventrations, les infections de prothèses sont décrites aussi bien après chirurgie ouverte que lors d'un abord coelioscopique. Ce taux est de 6% à 10% pour la voie ouverte et de 0% à 3,6% pour la voie laparoscopique. Ces infections du matériel prothétique sont des pathologies graves pour les patients et coûteuses pour le système de santé. En effet 4,5% des prothèses enlevées le sont pour infection et dans ces cas, les patients doivent subir en moyenne 2 procédures opératoires pour une prothèse enlevée (i.e ablation de la prothèse infectée et ensuite intervention pour cure de hernie avec une prothèse « biologique »)

La voie d'abord en chirurgie pariétale est aussi prise en compte dans l'analyse du taux d'infection. Une étude de l'European Hernia Society en 2001 (Korenkov m, Paul A, Sauerland S, et al. Classification and surgical treatment of incisional hernia: Results of an expert's meeting. Langenbecks Arch Surg 2001; 386: 65-73) montrait que lors du traitement de ces infections de matériel prothétique, il était plus souvent nécessaire d'enlever le matériel dans 0 à 7 % des cas, lorsque celui-ci était mis en place lors d'un abord coelioscopique, alors qu'il était nécessaire d'enlever le matériel uniquement dans 0 à 4% lors des abords par voie ouverte.

Le diagnostic d'une infection prothétique peut prendre différentes formes depuis un simple érythème cicatriciel jusqu'au sepsis grave et peuvent être classées en infections précoces et infections « tardives » en raison de leur étiologie probable. Les infections précoces sont dues le plus souvent, à une infection cutanée superficielle ou une fistule digestive alors que les infections chroniques surviennent sans fistule digestive associée. Les infections prothétiques précoces doivent être distinguées des réelles infections cutanées superficielles qui peuvent parfois être traitées par une antibiothérapie lorsque l'unique manifestation clinique est un aspect de cellulite. Ainsi le diagnostic Clinique d'une infection prothétique précoce est difficile, un sérome peut être confondu avec une collection en avant de la prothèse. Le diagnostic d'une infection associe des signes généraux communs à toutes les techniques chirurgicales : douleur, abcès péri cicatriciels ou péri prothétiques, fièvre, hyperleucocytose, augmentation des protéines de l'inflammation et de la vitesse de sédimentation, à des signes locaux associés à la technique chirurgicale. La présence d'un écoulement purulent par un orifice de drainage après une chirurgie ouverte ou par un ou plusieurs orifices de trocart après coelioscopie doit faire suspecter une infection. Certains patients peuvent présenter des infections prothétiques tardives avec suppuration persistante, cela semble survenir lorsqu'il existe formation d'un abcès sans signes de sepsis. Aussi le diagnostic certain d'une infection prothétique ne peut parfois se faire qu'après culture du liquide abcédé péri prothétique ou après culture de la prothèse elle-même.

Du point de vue microbiologique, 81% des infections de prothèses sont liées à Staph. Aureus dont 52% sont méthicilline-résistants et 17% de germes gram négatifs sont impliqués dans l'infection. L'infection du matériel prothétique est associée à plusieurs causes associant le patient (l'obésité, un traitement corticoïde au long court, le tabac...), le type de défect pariétal (récidive,...), la technique (durée opératoire prolongée,...) et la prothèse elle-même. La prothèse idéale serait composée d'un matériel inerte, qui induirait une réponse inflammatoire minime, qui favoriserait la colonisation vasculaire et fibroblastique en évitant l'encapsulation du matériel et son érosion. Elle devrait de plus s'intégrer dans les tissus voisins et limiter l'infection. Les prothèses pariétales fabriquées sous forme d'un filet («mesh») ont actuellement la structure d'un textile fait de fibres mono-ou multibrins tissées, tricotées ou collées à leurs intersections. Les textiles prothétiques sont caractérisés par la taille des pores qu'ils présentent. Ils sont fabriquées à partir de fibres non résorbables de polypropylène, de polyesters saturés à bas poids moléculaire (Dacron), ou de PTFE expansé. Certaines autres prothèses sont fabriquées à partir de fibres à résorption lente type Vicryl®.

Dès 1967, il a été montré que les sutures pariétales en milieu septique étaient de meilleures qualités avec des sutures mettant en oeuvre des fils monofilamentaires plutôt que des fils multifilamentaires ou filés de fibres. L'explication de ce constat intervient dix ans plus tard avec la mise en évidence de la propagation cinq à huit fois plus importante des bactéries dans les sutures réalisées à l'aide de fils multifilamentaires ou filés de fibres. Enfin dans les années 80, il est montré que les bactéries infectant les prothèses sont protégées de la phagocytose macrophagique dans les fils de suture multifilamentaires : les micropores ne permettant pas le passage cellulaire. Cette porosité liée aux filaments constitutifs de la prothèse peut aussi être liée au mode de mise en oeuvre des fils (tricotage ou tissage par exemple). Ainsi, la demanderesse a constaté que bien que formée de fils monofilamentaires, une prothèse tricotée à mailles serrées, sera résistante mécaniquement mais la colonisation cellulaire y sera plus difficile : ce qui diminuera l'intégration de la prothèse et augmentera en théorie le risque infectieux. Ainsi il existerait un risque infectieux plus important avec les prothèses formées de fils multifilamentaires ou filés de fibres et dont la mise en forme serait un maillage serré. Il a également été observé, dans le traitement des éventrations, que l'utilisation de prothèses de grande taille (300 cm² versus 200 cm²) est un facteur de risque infectieux. Le traitement des prothèses infectées comprend l'ablation du matériel prothétique implanté.

Il existe plusieurs classifications des types de prothèse. Selon la classification de Amid, les prothèses implantables sont classés en quatre groupes basés sur la taille des pores et le type de fibre constituant la prothèse, lesquels paramètres sont importants dans la prise en compte du risque infectieux. La masse surfacique de la prothèse peut également impacter le risque infectieux. Le type I correspond aux prothèses macroporeuses comprenant des pores de diamètre supérieur à 75 microns autorisant ainsi le passage des macrophages, des fibroblastes, des néovaisseaux et des fibres collagènes sans constitution d'une gaine fibre d'encapsulation et d'exclusion. Dans ce groupe se trouvent les différentes prothèses monofilamentaires à base de polypropylène (Marlex®, Prolène®, Surgipro®, Biomesh®).Le type II correspond à des prothèses microporeuses avec des pores < 10µ. Cette porosité semble insuffisante car elle ne permet pas la pénétration et l'adhésion cellulaire précédant la colonisation du matériau. Ces prothèses sont essentiellement fabriquées à base de ePTFE (Gore-tex®). Le type III correspond à des prothèses macroporeuses faites de multifilaments, type Mersilène®. Enfin, le type IV correspond à des prothèses imperméables faites par exemple de silastic ou équivalent.

Depuis quelques années, les prothèses dites « biologiques » sont utilisées. Ces prothèses permettent par leur incorporation biologique de créer un nouveau tissu pariétal comparable en fonction et en histologie au tissu de l'hôte. Elles sont constituées par une matrice acellulaire. Ces prothèses ont fait la preuve de leur utilité en chirurgie pariétale.

Ces prothèses « biologique » peuvent avoir deux origines :
- Animale : sous muqueuse d'intestin grêle porcin (Surgisis Cook, Bloomington, Indiana), derme porcin (Permacol, Covidien ; Collamend, Bard/Davol), Stratice, LifeCell, XenMatrix, Brennan Medical), derme de foetus bovin (Surgimend, TEL Bioscience), péricarde Bovin (Tutopatch, Tutogen Medical ; Véritas, Synovis); ou
- Humaine : derme humain (Alloderm, Lifecell, AlloMax, Bard/Davol, FlexHD, MTV, Tutomesh, Tutogen Medical).

Ces prothèses dites « biologiques », présentent des propriétés spécifiques en particulier dans le domaine de l'infection. Elles ne serviraient pas de « foyer » d'infection, ne « favoriseraient » pas l'infection et permettraient d'éviter le retrait du matériel si l'antibiothérapie contrôle l'infection. Ainsi ce type de matériel prothétique permettrait de traiter un défect pariétal y compris en milieu septique et de créer un nouveau tissu pariétal. Ces prothèses ont cependant pour inconvénients d'être couteuses et leur efficacité à long terme dans des cas complexes d'infection est encore inconnue. De plus, une étude (Shah BC, Tiwari MM, Goede MR, Eichler MJ, Hollins RR, McBride CL, Thompson JS, Oleynikov D. Not all biologics are equal! Hernia. 2011 Apr;15(2):165-71. Epub 2010 Dec 28 Shah), portant sur 58 patients, traités par "bioprothèse" suite à une éventration infectée, entre 2002 et 2007, fait état à un an de récidives de hernies pour 27.9% de la population testée ainsi que la nécessaire ablation des bioprothèses suite à une infection. Ces prothèses biologiques recommandées pour être mises en place sur des sites infectés sont trop épaisses (de l'ordre de 1 mm), et ont le désavantage de ne pas être transparentes. En outre, elles nécessitent de moyens de traitement complexe de la matière première d'origine animale ou humaine afin d'éliminer toutes la protéine de la matrice collagène qui les composent. De ce fait elles sont proposées sur le marché à des prix très élevées. De plus, l'origine animale ou humaine pourrait également dans l'avenir poser à nouveau des problèmes sanitaires sérieux, tel qu'on a pu le connaître dans la passé avec par exemple la maladie de Creutzfeldt-Jakob (ou encéphalopathie spongiforme subaiguë).

Il a également été observé, dans le traitement des éventrations, que l'utilisation de prothèses de grande taille (300 cm² versus 200 cm²) est un facteur de risque infectieux. Le traitement des prothèses infectées comprend l'ablation du matériel prothétique implanté.

Dans un travail publié en 2007 dans Hernia (Champault G, Bernanrd C, Rizk N etal. Inguinal hernia repair : the choice of prosthesis outweighs that of technique. Hernia 2007 ; 11 :125-128) l'équipe de Gérard Champault titrait que dans le cadre de la réparation des hernies de l'aine le choix de la prothèse est plus important que le choix de la technique. Le choix du matériel prothétique est donc crucial dans la gestion du risque infectieux.

Il existe ainsi un besoin pour une prothèse implantable palliant les problèmes précités limitant le risque infectieux, pouvant être implantée en première intention en traitement d'une hernie afin d'apporter le renfort mécanique nécessaire tout en limitant les risques infectieux, ou en seconde intention, en étant appliquée contre une prothèse déjà implantée afin de traiter une infection et renforcer le soutien apporté.

La présente invention cherche ainsi à proposer une prothèse implantable simple à fabriquer, à un cout raisonnable, et qui ne comprennent pas comme moyen de renfort mécanique, un composant d'origine animale ou humaine.

### Objet et résumé de l'invention

La présente invention a pour objet, selon un premier aspect, une prothèse implantable, notamment pour la réfection des hernies, comprenant avantageusement une pièce textile poreuse comprenant des fils monofilamentaires et ayant au moins une première face, en particulier ayant des première et seconde faces opposées, et au moins un revêtement fonctionnel, continu ou discontinu, disposé selon tout ou partie de ladite première face. Ledit revêtement est dans une composition comprenant un miel ou un mélange de plusieurs miels représentant au moins 50% en masse de la masse totale de ladite composition, et en ce que la masse surfacique de la pièce textile sans revêtement est inférieure ou égale à 150 g/m².

La combinaison de la pièce textile poreuse selon l'invention et d'un revêtement fonctionnel à base de miel(s) permet une colonisation harmonieuse de la pièce textile par des microcapillaires et les bourgeons fibroblastiques, sans constitution d'une gaine fibreuse d'exclusion et d'encapsulation, limitant ainsi les risques infectieux et donc la récidive opératoire.

Le miel, quel que soit son origine, contient de fortes quantités de sucres mais très peu d'eau. Ce dernier facteur empêche la prolifération bactérienne. De plus, l'osmolarité élevée du miel contribue à extraire l'eau contenue dans les bactéries ce qui a pour conséquence leur déshydratation et leur élimination.

Un miel ou un mélange de miels dilué reste actif face aux bactéries grâce à la production de peroxyde d'hydrogène en présence d'eau du fait de l'activation d'une enzyme glucose-oxydase secrétée par les abeilles. Cette enzyme a pour rôle d'oxyder le glucose en acide gluconique et corrélativement de générer du peroxyde d'hydrogène. Le peroxyde d'hydrogène est alors le composant principal responsable de l'activité antiseptique et antibactérienne du miel. Le peroxyde d'hydrogène empêche les bactéries de se développer au sein du miel comprenant de l'eau, en attendant que celui-ci atteigne son taux en sucres optimal. La glucose-oxydase, sécrétée par la glande hypopharyngée de l'abeille, participe également à la transformation du nectar en miel. Dans beaucoup de cas, l'activité du peroxyde d'hydrogène issu de la transformation du miel peut être inhibée facilement par la chaleur ou par la présence d'une catalase. Pourtant, on a pu constater que le miel conserve son activité antimicrobienne malgré la présence de cette catalase. En effet d'autres constituants composant le miel tels que le méthyl syringate ou le methylglyoxal, interviennent pour expliquer la persistance de cette activité antimicrobienne malgré l'inhibition du peroxyde d'hydrogène. Etant donné que l'activité antibactérienne est multi factorielle, le miel peut donc inhiber la croissance d'un large spectre de bactéries, champignons, protozoaires et virus sans que ces derniers ne puissent développer de résistance.

En plus de ses propriétés antimicrobiennes, le miel peut endiguer l'infection de plusieurs manières : en stimulant le système immunitaire ou par une action anti-inflammatoire et anti-oxydante. La prolifération des lymphocytes B et T dans le sang ainsi que l'activation des phagocytes est stimulée par le miel à des concentrations de 0,1% (en masse). A une concentration de 1% (en masse), le miel peut stimuler les monocytes à sécréter des cytokines, du TNF, de l'IL-1 et de l'IL-6 qui activent la réponse immunitaire contre l'infection.

Par son effet anti-inflammatoire, le miel réduit les oedèmes et diminue l'exsudation. Par conséquent, il réduit également la douleur qui provient de l'excitation des terminaisons nerveuses par les prostaglandines libérées lors du processus inflammatoire et de l'oedème qui exerce une pression sur les nerfs.

Le miel agit également sur le processus de cicatrisation. Les modèles animaux ont démontré que le miel accélérait la cicatrisation en agissant sur l'angiogénèse, la granulation et l'épithélialisation. L'hypothèse serait que le miel agit en déclenchant la cascade cellulaire de l'inflammation qui aboutit à la production de facteurs de croissance. Ces derniers contrôlent alors l'angiogénèse, et la prolifération des fibroblastes et des cellules épithéliales. D'ailleurs, des études récentes ont montré que le miel stimulait la production des cytokines de l'inflammation (ex TNFα, IL6, IL1β) par les macrophages, via le Toll-like réceptor 4 (TLR4).

La composition pour la formation du revêtement selon l'invention doit être cytocompatibile.

Ledit au moins un miel ou mélange de plusieurs miels présente naturellement un pH faible, c'est-à-dire compris entre 3,4 et 5,5. En effet, ledit au moins un miel ou mélange de miels comprend de l'acide gluconique, et éventuellement de l'acide citrique, de l'acide formique, de l'acide lactique, de l'acide oxalique et de l'acide succinique. D'autres acides sont également présents, combinés sous forme de lactones.

Il est donc nécessaire que la composition est un pH propre à son implantation, en particulier neutre (c'est-à-dire de l'ordre de 7). Ceci peut être obtenu en diluant ledit au moins un miel ou le mélange de miels et/ou en ajoutant un agent de neutralisation à ladite composition afin d'atteindre le pH désiré ou encore en utilisant du miel de Bourdaine mono floral pur, dont le pH oscille entre 5 et 6,5, voir 7 (proche de la neutralité).

Les miels sont généralement visqueux allant du jaune très pâle (presque blanchâtre) jusqu'au brun foncé. Suite à toutes les étapes de son élaboration, faisant intervenir une multitude d'acteurs, tant animaux que végétaux, il n'existe pas un miel mais une réelle diversité des miels, les possibilités de variabilité étant présentes à chaque étape. Les caractéristiques physico-chimiques, la composition seront différentes d'un miel à l'autre. Tous les constituants du miel ne sont pas encore connus.

Dans un mode de réalisation, la proportion massique en eau dans ledit au moins un miel ou ledit mélange de plusieurs miels est supérieure ou égale à 10%, en particulier inférieure ou égale à 30%, plus particulièrement compris entre 14% et 25%, notamment de l'ordre de 17%.

Le miel contient des grains de pollen, qui après identification permettront de définir son origine florale et donc géobotanique. En comparaison, un miel issu d'une ruche nourrie au sirop de sucre, ne contiendra que très peu voire pas du tout de grains de pollen puisque les abeilles, dans ce cas, n'iront pas butiner et ne rapporteront pas de pollen. La présence de grains de pollen présagera de la qualité du miel mais leur absence pourra être originaire d'une ultrafiltration.

Ledit au moins un miel ou un mélange de miels comprend des glucides, des protides, des lipides, et éventuellement des minéraux et/ou des vitamines.

En particulier, les glucides représentent plus de 90% en masse de la masse totale sèche dudit au moins un miel ou dudit mélange de miels.

On comprend dans le présent texte par masse sèche dudit au moins un miel ou dudit mélange de miels, que ledit au moins un miel ou mélange de miels comprend une proportion massique en eau inférieure ou égale à 1%.

Lesdits glucides présents dans ledit au moins un miel ou mélange de miels comprennent des monosaccharides ou oses (fructose, glucose), des oligosaccharides (polymères d'oses comprenant de 2 à 10 unités d'oses, par exemple le saccharose et le maltose ou encore l'iso-maltose, le maltotriose, le turanose), et des polysaccharides ou polyosides. Les polyosides comprennent plus de de dix unités d'oses, et sont ainsi considérés comme des sucres dits lents car plus longs à métaboliser (tels que l'amidon, la cellulose et l'hémicellulose). Les glucides peuvent comprendre également le nigériose, le kojibiose, le leucrose, le mélézitose, l'erlose, le kestose, le raffinose et le dextrantriose.

En particulier, les protides représentent au plus 1% en masse de la masse totale sèche dudit au moins un miel ou dudit mélange de miels. Les protides comprennent notamment des protéines, des peptides (telle que la défensin-1) et des acides aminés.

Lors de la synthèse du miel par les abeilles, leurs enzymes convertissent le saccharose en glucose et en fructose, et la glucose-oxydase convertit le glucose en peroxyde d'hydrogène et en acide gluconique. Ces deux derniers composés sont présents pour empêcher la détérioration du miel non mûr par les bactéries.

Le miel ou le mélange de miels, lorsqu'ils sont naturels, comprend de la proline et de l'hydroxyproline provenant de la salive de l'abeille mais également de l'alanine, de l'acide glutamique, de la glycine et de la leucine.

Les lipides comprennent des stérols sous forme de cholestérol libre ou d'esters de cholestérol, ainsi que des triglycérides et des acides gras libres. Ils sont présents à l'état de traces dans la plupart des miels, le miel de tournesol en étant le plus riche.

La teneur massique en minéraux dans ledit miel ou mélange de miels dépendent des nombreux facteurs, dont l'origine géobotanique du miel (c'est-à-dire de l'espèce de l'abeille et du type de sol) mais également du produit récolté pour sa confection, un miel de miellat sera en moyenne près de deux fois plus concentré en minéraux qu'un miel de nectar. Plus un miel est foncé, plus il sera riche en minéraux. Un miel moyen contient 0,26 % de minéraux en masse par rapport à sa masse sèche dont environ la moitié est représentée par le potassium avec 100 à 2000 µg/g, pour le reste, plus d'une trentaine d'oligoéléments sont retrouvés : le phosphore avec 25 à 145 µg/g, le calcium avec 12 à 90 µg/g, le soufre avec 7 à 67 µg/g, le magnésium avec 4 à 55 µg/g, le manganèse avec 0,2 à 10 µg/g et le silicium, le bore, le fer, le zinc, le cuivre, le baryum avec des concentrations inférieures à 10 µg/g.

Ledit au moins un miel ou mélange de miels peut comprendre un plusieurs enzyme(s). Les enzymes peuvent être d'origine animale (apportée par la salive des abeilles), et végétale (dans ce cas, elles proviennent du nectar).

Les enzymes d'origine animale sont choisis parmi : l'invertase (participe à l'hydrolyse du saccharose pour la formation du fructose et du glucose), l'amylase (participe à l'hydrolyse de l'amidon en maltose puis en glucose), la glucose-oxydase (participe à l'oxydation du glucose en acide gluconique, libérant du peroxyde d'hydrogène). La glucose-oxydase est également présente dans la gelée royale.

La glucose-oxydase est très sensible à la lumière et n'est active que pour une teneur massique en eau supérieure à 17% dans le miel ou un mélange de miels.

Les enzymes d'origine animale sont choisis parmi : la catalase (responsable la égradation du peroxyde d'hydrogène), et les phosphatases acides.

La ou les vitamines peut/peuvent être : des vitamines hydrosolubles du groupe B, telles que B1, B2, B3, B5, B6, B8 et B9 ; les vitamines liposolubles A, D, E et K. Ces vitamines se retrouvent à l'état de traces dans les grains de pollen et sont donc quasiment indétectables dans le miel. La vitamine C est présente à l'état de traces également, certains miel en sont plus fournis, par exemple dans le miel de menthe. Les vitamines sont apportées par les grains de pollen principalement.

Ledit au moins un miel ou mélange de miels comprend un ou plusieurs pigment(s) qui peut/peuvent être : des caroténoïdes et des flavonoïdes (0,006%).

Ledit au moins un miel ou mélange de miels peut également comprendre des polyphénols, représentés par les flavonoïdes qui ont une activité antimicrobienne, également antioxydante très puissante.

Ledit au moins un miel ou mélange de miels peut également comprendre de l'hydroxyméthylfurfural qui est un dérivé du fructose traduisant la qualité du miel et de sa conservation. En quantité élevée, il révèle un stockage de longue durée, une dénaturation par exposition à la chaleur ou un ajout d'un mélange de glucose et fructose.

Ledit au moins un miel ou mélange de miels peut également comprendre un facteur glykutile. Ce facteur intervient dans l'utilisation du glucose par l'organisme. Il favorise d'autre part la fixation du calcium et de magnésium par les cellules. Il est issu des sécrétions des glandes salivaires de l'abeille.

Ledit au moins un miel ou mélange de miels peut également comprendre des alcools et des phénols, lesquels ont une activité bactériostatique, et bactéricide.

La prothèse implantable ne comprend pas de matière biologique type tissus d'origine animale ou humaine, au sens des prothèses biologiques décrites ci-dessus dans l'art antérieur limitant ainsi son coût de fabrication et simplifiant son procédé de fabrication. Les paramètres combinés en termes de matériaux employés, c'est-à-dire fils monofilamentaires et revêtement à base de miel(s), ainsi qu'une faible masse surfacique pour la pièce textile, limitent les risques de réactions négatives chez le patient.

Le revêtement fonctionnel à base de miel(s) est efficace contre les infections, est biorésorbable, et ne sera pas le foyer d'une infection supplémentaire.

La pièce textile selon l'invention peut comprendre un ou plusieurs textile(s) tricoté(s) et/ou tissé(s), de préférence il s'agit d'une pièce textile tricotée, c'est-à-dire comprenant un ou plusieurs textile(s) tricoté(s), en particulier comprenant un seul textile tricoté.

Le ou les textile(s) tricoté(s) est/sont à mailles jetées ou à mailles cueillies, et de préférence à mailles jetées, par exemple tricoté(s) sur un métier à tricoter chaine, tel qu'un métier Raschel. Un tricot à mailles jetées présentera moins d'élasticité qu'un tricot à mailles cueillies mais de meilleures performances mécaniques en tant que renfort mécanique.

La pièce textile selon l'invention peut comprendre également des fils multifilamentaires et/ou des fils filés de fibres, mais de préférence est constituée de fils monofilamentaires.

On comprend par première ou seconde face de la pièce textile, toute face interne ou externe de ladite pièce qu'elle soit plane ou en trois dimensions.

La pièce textile selon l'invention peut être en trois dimensions ou plane (les première et seconde faces sont dans des plans sensiblement parallèles). Lorsque la pièce textile est en trois dimensions, elle peut avoir la forme d'un cône ou comprendre une protubérance s'étendant de la première ou seconde face. Dans ce cas, une face correspond à la surface interne ou externe de la pièce textile.

On comprend par « pièce textile poreuse » que ladite pièce textile comprend des pores ou jours débouchant selon sa première face, et éventuellement selon sa seconde face, opposée à ladite première face, et s'étendant donc sur toute son épaisseur. En particulier, la pièce textile est macroporeuse et comprend des pores dont la taille moyenne (calculée sur 10 mesures) est supérieure à 75 µm, de préférence de taille moyenne (calculée sur 10 mesures) supérieure ou égale à 1 mm.

Le revêtement fonctionnel selon l'invention peut être continu, c'est-à-dire sous forme de film appliqué sur tout ou partie de la première face, ou, discontinu, c'est-à-dire sous forme de motifs (points, bandes, ou autres formes) sur tout ou partie de la première face.

L'utilisation d'un miel ou d'un mélange de plusieurs miels permet d'obtenir un revêtement fonctionnel ayant une activité antibactérienne sur un large spectre bactérien et ainsi de pouvoir traiter le site chirurgical infecté.

La pièce textile peut être résorbable (c'est-à-dire totalement dégradée par l'organisme au terme de 30 jours à compter de l'implantation), semi-résorbable, ou non résorbable (c'est-à-dire restant non dégradée par l'organisme une fois implanté). Lorsque la pièce textile est semi-résorbable, elle comprend une partie résorbable et une partie non résorbable, en particulier la proportion massique de la partie résorbable comparativement à la masse totale de la pièce textile (sans revêtement) est supérieure ou égale à 10% et inférieure ou égale à 90%, plus particulièrement supérieure ou égale à 40% et inférieure ou égale à 60%. Dans ce dernier cas, la proportion massique de la partie non résorbable comparativement à la masse totale de la pièce textile (sans revêtement) est supérieure ou égale à 10% et inférieure ou égale à 90%, plus particulièrement supérieure ou égale à 40% et inférieure ou égale à 60%.

La pièce textile a de préférence une porosité de type I selon la classification d'AMID (Amid P. Classification of biomaterials and their related complications in abdominal wall surgery. Hernia. 1997; 1(1):15-21).

De préférence, la pièce textile est transparente, c'est-à-dire qu'il est possible de visualiser ce qui est placé face à la seconde face lorsque l'on regarde la première face, et inversement.

De préférence, la masse surfacique de la pièce textile (sans revêtement) est supérieure ou égale à 30 g/m², encore de préférence inférieure ou égale à 100 g/m², plus préférentiellement inférieure ou égale à 80 g/m², en particulier inférieure ou égale à 50 g/m².

Le miel ou les miels est/sont choisi(s) parmi les miels monofloraux et/ou multifloraux, de préférence monofloraux (afin de maîtriser les proportions massiques en defensin-1 et en méthylglyoxal définis ci-dessous).

Le miel ou les miels employés est/sont d'origine naturelle ou artificielle, de préférence d'origine naturelle.

Dans un mode de réalisation, le miel ou le mélange de plusieurs miels représente(nt) au moins 70% en masse de la masse totale (g) de ladite composition, de préférence au moins 85% en masse de la masse totale (g) de ladite composition, en particulier au moins 90% en masse de la masse totale (g) de ladite composition, plus particulièrement au moins 95% en masse de la masse totale (g) de ladite composition.

Ladite composition peut comprendre un ou plusieurs agent(s) texturant(s) biocompatible(s) ou un ou plusieurs principe(s) actif(s) également biocompatible(s).

Dans une variante, ledit miel ou le mélange de plusieurs miels représente au moins 80% en masse de la masse totale de ladite composition.

Dans une variante, ladite composition comprend au moins 0,5 µg (micro gramme) de defensin-1 pour un gramme dudit au moins un miel ou dudit au moins un mélange de plusieurs miels.

La defensin-1 est un peptide cationique ayant des propriétés antimicrobiennes. La gelée royale comprend une proportion massique en defensin-1 plus élevée que celle présente dans le miel.

Le miel naturel comprend de la defensin-1 dont la proportion massique dépend du type de miel. La défensin-1 peut ainsi être apportée par le miel ou le mélange de plusieurs miels et/ou apportée séparément en tant que principe actif.

Dans une variante, ladite composition comprend au plus 100 mg (milligramme) de méthylglyoxal pour un kilogramme dudit au moins un miel ou dudit au moins un mélange de plusieurs miels.

Le miel naturel comprend du méthylglyoxal dont la proportion massique dépend du type de miel. Le méthylglyoxal peut ainsi être apporté par le miel ou le mélange de plusieurs miels et/ou apportée séparément en tant que principe actif.

Les proportions massiques en defensin-1 et en méthylglyoxal ont été déterminées par les inventeurs afin d'obtenir une activité bactéricide suffisante sans susciter d'intolérance de l'organisme lors de l'implantation, en particulier si la proportion massique en méthylglyoxal ne dépasse pas le seuil indiqué ci-dessous.

Le méthylglyoxal est produit à partir du dihydroxyacétone synthétisé par certaines plantes notamment le manuka. Dans certaines conditions, c'est-à-dire à 37°C, ce composé est transformé en méthylglyoxal. Il est peu présent dans le miel mais après une incubation (dans la ruche ou de manière artificielle) à 37°C, son taux va augmenter. Il a la capacité de se fixer aisément à de nombreuses molécules : Protéines, lipides, et ADN. Il est donc hautement réactif.

En utilisation dans le miel, ces effets néfastes ne sont a priori pas observés, puisqu'ils sont compensés par les nombreux antioxydants qui composent le miel. Il est immuno-modulateur en stimulant la production de TNFα et y (Tumor Necrosis Factor), et jouerait ainsi un rôle dans la cicatrisation. C'est un agent extrêmement oxydable, il a une structure chimique très instable et il peut produire des oxydations entraînant des dommages au niveau de l'ADN.

Dans une variante, ledit revêtement représente au moins 50%, de préférence au moins 60%, encore de préférence au moins 70%, en masse de la masse totale de la pièce textile comprenant ledit revêtement.

Dans une variante, ledit miel ou ledit mélange de plusieurs miels est/sont choisis dans la liste comprenant : le miel monofloral de trèfle, le miel monofloral de vipérine, le miel monofloral d'acacias ou leur mélange, en particulier le miel de manuka est exclu de ladite liste.

Les miels cités dans la liste ci-dessus sont préférés car ils comprennent une proportion massique en defensin-1 élevée, tout en limitant la proportion massique en méthylglyoxal.

De préférence, ladite composition comprend alors au moins 0,7 µg de defensin-1 pour un gramme dudit au moins un miel ou dudit au moins un mélange de plusieurs miels, et au plus 40 mg de méthyglyoxal par kilogramme dudit au moins un miel ou dudit au moins un mélange de plusieurs miels.

Dans une variante, ladite composition comprend de la gelée royale, en particulier d'Apis Mellifera.

De préférence, la proportion massique en gelée royale dans la composition est supérieure à 0% et inférieure ou égale à 15%, encore de préférence inférieure ou égale à 10%, plus préférentiellement inférieure ou égale à 5%.

La gelée royale comprend naturellement une proportion massique en defensin-1 supérieure à celle d'un miel naturel. L'utilisation de gelée royale permet ainsi d'augmenter significativement la proportion massique en defensin-1 dans la composition, et donc d'améliorer les propriétés bactéricides du revêtement.

Dans une variante, ladite composition comprend au moins un polymère biocompatible liquide à une température supérieure ou égale à 10°C et à une température inférieure ou égale à 70°C (afin de ne pas dégrader les propriétés du ou des miel(s)), notamment le polycaprolactone et/ou le polymère d'acide lactique de forme D (PDLA).

Avantageusement, le polymère d'acide lactique de forme D (PDLA) est liquide à une température inférieure ou égale à sa température de transition vitreuse (Tg) qui est de l'ordre de 60°C.

De préférence, la proportion massique en dit au moins un polymère biocompatible par rapport à la masse totale de ladite composition est inférieure ou égale à 20%, en particulier inférieure ou égale à 10%, plus particulièrement inférieure ou égale à 5%.

Si ledit au moins un miel ou ledit mélange de miels est trop liquide à température ambiante (c'est-à-dire à une température comprise entre 15°C et 35°C, en particulier entre 15°C et 30°C), l'ajout dudit au moins un polymère biocompatible permet d'ajuster la viscosité de la composition afin que la composition une fois disposée sur la première face de la pièce textile ne coule pas de cette dernière, du moins le temps que cette dernière soit fabriquée et emballée en vue sa stérilisation.

Ledit au moins un polymère biocompatible peut être résorbable, non résorbable ou semi résorbable, et de préférence résorbable.

Dans une variante, ladite pièce textile sans revêtement a une masse surfacique inférieure ou égale à 80 g/m².

Dans une variante, le miel ou ledit mélange de plusieurs miels est/sont un ou des miel(s) d'origine naturelle et/ou artificielle.

Dans une variante, le miel ou les miels artificiel(s) est/sont constitué(s) majoritairement de glucose, de fructose et de saccharose.

Dans une variante, la pièce textile est une pièce tricotée à partir de fils monofilamentaires, en particulier ayant un diamètre inférieur ou égal à 0,2 mm.

Dans une variante, la pièce textile est une pièce tricotée comprenant des mailles ouvertes et des mailles fermées et en ce que la proportion en nombre de mailles ouvertes par rapport au nombre total de mailles ouvertes et de mailles fermées pour une pièce textile de 100 cm² (10 cm * 10 cm) est supérieure ou égale à 50%, encore de préférence supérieure ou égale à 80%.

Les inventeurs ont pu observer qu'une proportion élevée en mailles ouvertes permettait de limiter les infections bactériennes et corrélativement permettait d'emporter davantage de composition pour la formation du revêtement fonctionnel antibactérien.

Dans une variante, les fils monofilamentaires de la pièce textile sont dans un ou plusieurs matériaux polymère(s) synthétique(s) biocompatible(s), en particulier choisi(s) dans la liste comprenant : les polyoléfines, et notamment le polypropylène et le polyéthylène ; les polyesters, et notamment le polyéthylène téréphtalate ; les polyamides, et notamment le polyamide 6-6 ; les polymères d'acide lactique, et notamment l'acide polylactique de forme L ou D ou leur mélange (PLLA, PLA, PDLA), ... ; encore de préférence il s'agit du polypropylène.

La présente invention a pour objet, selon un deuxième aspect, un procédé de fabrication d'une prothèse implantable selon l'une quelconque des variantes de réalisation définies ci-dessus, comprenant les étapes suivantes :
(i) fourniture d'une pièce textile poreuse ayant au moins une première face, en particulier ayant des première et seconde faces opposées, et d'une composition biocompatible comprenant un miel ou un mélange de miels ;
(ii) application de ladite composition à l'état liquide, éventuellement préalablement chauffée à une température supérieure ou égale à 25°C, sur au moins ladite première face, en sorte de former un revêtement fonctionnel, continu ou discontinu;
(iii) optionnellement refroidissement de ladite pièce textile revêtue ;
(iv) stérilisation de ladite pièce textile revêtue.

La température du revêtement selon l'invention lors de son application selon tout ou partie de la première face de la pièce textile est déterminée en sorte de ne pas dégrader la ou les propriété(s) dudit miel ou dudit au moins un mélange de miels, de préférence à une température inférieure ou égale à 35°C (température normale d'une ruche), en particulier afin de ne pas dégrader la ou les enzyme(s) présente(s).

Avantageusement, l'étape de stérilisation, en particulier l'application de rayons gamma, notamment une dose supérieure ou égale à 25 kGy (i.e 25 000 Grays) ou plus, ne détruit pas la ou les enzyme(s) présente(s).

De même, la composition selon l'invention est de préférence préparée en ne portant pas ledit au moins un miel ou mélange de miels à une température dégradant les propriétés dudit miel ou dudit au moins un mélange de miels, en particulier la ou les enzyme(s) présente(s).

Dans une variante, la pièce textile est plane, et les première et seconde faces sont opposées et dans des plans sensiblement parallèles, et en ce que ladite composition est appliquée sous forme d'un film mince selon au moins ladite première face à l'aide d'une filière d'extrusion comprenant au moins une fente longitudinale d'extrusion de ladite composition, en particulier de forme rectangulaire, ayant un axe longitudinal L, ladite filière se déplaçant au-dessus de la pièce textile selon une direction T transversale à l'axe longitudinal L.

Dans une variante, ladite fente longitudinale d'extrusion présente une largeur (l1) et une longueur (L1), la largeur l1 étant supérieure à 0 mm et inférieure ou égale à 1 mm, de préférence inférieure ou égale à 500µm, encore de préférence inférieure ou égale à 250 µm, encore plus préférentiellement inférieure ou égale à 150µm.

Dans une variante, le miel ou le mélange de plusieurs miels a subit une étape de filtration pour ôter les impuretés solides et une étape de stérilisation avant l'étape (ii), notamment par exposition à des rayons gamma (25 KGy, 25 kilogray).

### Brève description des dessins

L'invention sera mieux comprise à la lecture de la description qui suit des trois modes de réalisation de l'invention donnés à titre d'exemple non limitatif, en référence aux dessins annexés, sur lesquels :
- la figure **1** est une représentation schématique du schéma de mailles de la pièce tricotée d'un premier exemple de prothèse implantable selon l'invention ;
- la figure **2** est une représentation schématique, vue de côté, de l'étape d'application du procédé de fabrication selon l'invention ;
- la figure **3** est une représentation schématique, vue de face, de l'étape représentée à la figure **2** **;**
- la figure **4** est une représentation schématique du schéma de mailles de la pièce tricotée d'un seconde exemple de prothèse implantable selon l'invention ;
- la figure **5** est une représentation schématique, vue de côté, d'une variante du dispositif d'application d'au moins un solvant de solubilisation sous forme de film.

### Description détaillée des exemples

Le premier exemple de prothèse implantable **1,** en particulier pour la réfection des hernies, comprend une pièce textile poreuse **3** constituée d'un tricot de fils monofilamentaires en polypropylène de diamètre 0,15 mm qui est stabilisée (ou autrement dit thermofixée) sur rame. Ladite pièce textile **3** est plane et comprend une première face **3a** et une seconde face **3b.** La première face **3a** et la seconde face **3b** sont opposées et dans des plans sensiblement parallèles.

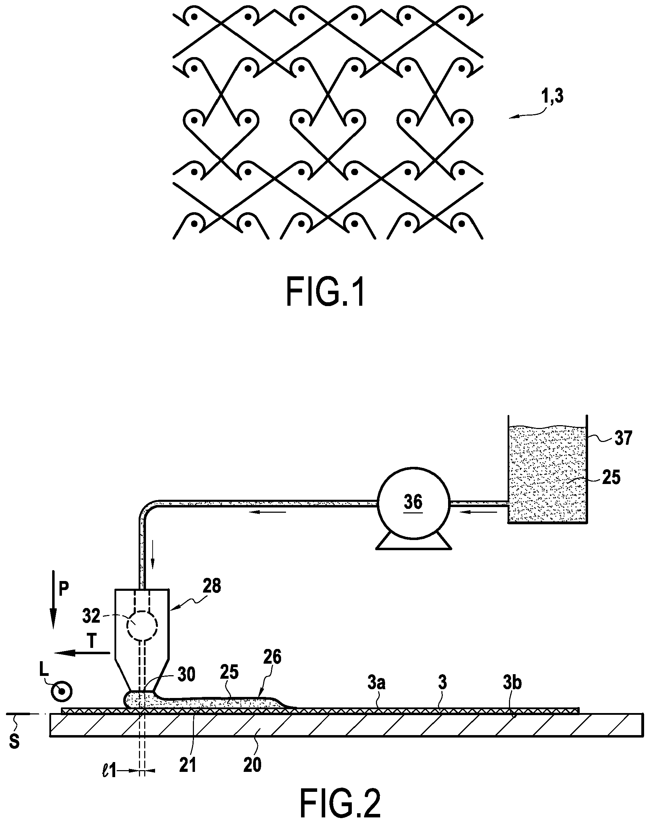

La pièce textile tricotée **3** est tricotée sur un métier à tricoter Rachel Karl Meyer comportant 20 aiguilles au pouce (un pouce est égal à 25,4 mm) selon le schéma de mailles représenté à la figure **1****.** La pièce textile tricotée **3** comprend uniquement des mailles ouvertes et à une masse surfacique de 75 g/m². Après l'étape de tricotage, le panneau textile tricoté obtenu en sortie du métier à tricoter subit une étape de traitement thermique à une température de l'ordre de 140°C sur une rame textile pendant au moins 60 secondes, puis le panneau textile tricoté, de 150 mm x 250 mm, est refroidi à température ambiante afin de le stabiliser dans une forme plane. La pièce textile tricotée **3** est découpée à l'aide d'un laser dans le panneau tricoté en sorte de présenter des dimensions de 150 mm * 150 mm.

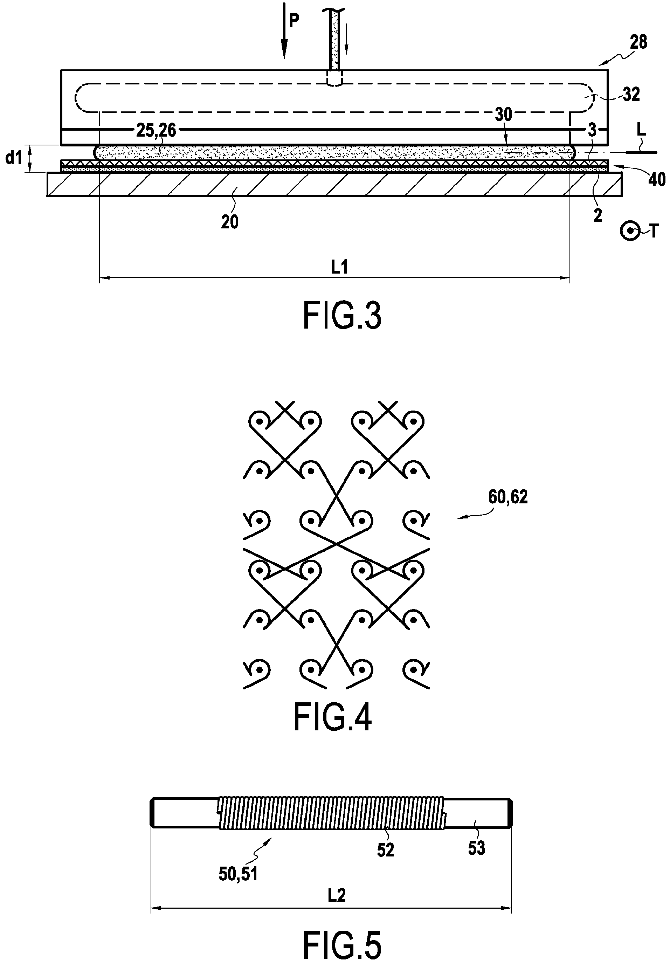

Les figures **2** et **3** représentent de manière schématique le mode de réalisation préféré du procédé de fabrication de la prothèse implantable **1**.

On dispose tout d'abord la pièce textile tricotée **3,** selon sa seconde face **3b** sur un support **20** parfaitement plan, en particulier dans une zone de réception **21** dudit support **20.** Ledit support **20** est dans cet exemple précis une plaque en verre.

On applique la composition à base de miel(s) **25,** dont la température est de l'ordre de la température ambiante, sur la première face **3a** de la pièce tricotée **3** à l'aide d'une filière d'extrusion **28.** Laquelle filière d'extrusion **28** comprend au moins une fente longitudinale d'extrusion **30** de ladite composition **25,** en particulier de forme rectangulaire, ayant un axe longitudinal **L.** La filière d'extrusion **28** se déplaçant au-dessus de la première face **3a** de la pièce tricotée **3** selon une direction **T** perpendiculaire à l'axe longitudinal **L.**

La viscosité de la composition **25** est déterminée en sorte que ladite composition **25** reste sur la première face **3a** de la pièce tricotée **3** et éventuellement migre dans son épaisseur mais pas selon la seconde face opposée **3b.**

La fente longitudinale d'extrusion **30** présente une largeur **l1** et une longueur **L1**. Dans un exemple précis, la largeur **l1** est d'environ 150 µm, et la longueur **L1** est d'environ 40 cm.

La fente de la filière d'extrusion **30** débouche au-dessus de la première face **3a** de la pièce tricotée **3,** et délivre un film **26** de composition **25** selon une direction **P** sensiblement perpendiculaire à son axe longitudinale **L** et au plan **S** comprenant la pièce tricotée **3.**

La distance **d1** entre ladite fente longitudinale d'extrusion **30** et le support **20** recevant ledit panneau **3** est de l'ordre de 400 µm.

La filière d'extrusion **28** est en liaison fluidique avec une pompe **36,** laquelle est en liaison fluidique avec une réserve principale **37** de ladite composition **25.**

Avantageusement, la filière d'extrusion **28** comprend une réserve **32** de ladite composition **25** alimentée régulièrement par la réserve principale **37.** La pompe **36** comprend avantageusement des moyens de dosage dans le temps de la quantité de composition pompée.

La vitesse de défilement de la filière d'extrusion **30** selon la direction **T,** le support étant fixe, au-dessus du panneau **3,** est comprise entre 0,1 cm/seconde et 10 cm/seconde.

La prothèse implantable **1** comprenant la pièce textile **3** et le revêtement issu du film **26** est ensuite placée dans un double sachet de stérilisation en vue d'être stérilisée à l'oxyde d'éthylène.

Dans cet exemple précis, la composition 25 est constituée de miel de vipérine (de masse volumique 1,422 grammes/cm3) qui a été, préalablement à son utilisation, filtré puis stérilisé aux rayonnements gamma à une dose de 25 KGy.

Le revêtement déposé forme un film continu **26** et présente une masse surfacique de l'ordre de 215 g/m².

Le second exemple de prothèse implantable **60** comprend une pièce textile tricotée **62** à partir de fils monofilamentaires en polypropylène de diamètre 0,1 mm qui est stabilisée (ou autrement dit thermofixée) sur rame. La pièce textile tricotée **62** est tricotée sur un métier à tricoter Rachel Karl Meyer comportant 20 aiguilles au pouce (un pouce est égal à 25,4 mm) selon le schéma de mailles représenté à la figure **4****.** La pièce textile tricotée **62** comprend avantageusement uniquement des mailles ouvertes et à une masse surfacique de 40 g/m². Après l'étape de tricotage, le panneau tricoté en sortie du métier à tricoter (ayant éventuellement subit une découpe préliminaire) subit un traitement thermique à 130°C sur une rame textile pendant au moins 60 secondes, puis est refroidie à température ambiante, afin de le stabiliser dans une forme plane. La pièce tricotée **62** est ensuite découpée sur une table, dans le panneau tricoté stabilisé, à l'aide d'un laser selon des dimensions 200 mm x 200 mm. La pièce textile tricotée **62** est ensuite placée sur une table d'enduction afin d'être enduite d'une composition à base de miel(s) à l'aide d'une barre avec fil enroulé de « Mayer » représentée à la figure **5****.** Le diamètre du fil **52** enroulé sur la barre **53** est de préférence compris entre 0,08 mm et 1,50 mm (bornes inférieure et supérieure incluses), en particulier de l'ordre de 1,00 mm. Le diamètre de la barre **53** est compris entre 6 mm et 30 mm (bornes inférieure et supérieure incluses). La longueur **L2** de la barre **53** pouvant être de l'ordre de 30 cm, voire de plusieurs mètres selon les besoins, et de préférence dans cet exemple précis de l'ordre de 40 cm.

Dans cet exemple précis, la composition est constituée de miel de trèfle (de masse volumique 1,40 grammes/cm3) qui a été, préalablement à son utilisation, filtré puis stérilisé aux rayonnements gamma à une dose de 25 KGy.

Le revêtement déposé forme un film continu et présente une masse surfacique de l'ordre de 100 g/m².

La prothèse implantable **60** comprenant la pièce textile **62** et le revêtement issu du film de composition déposée, est ensuite placée dans un double sachet de stérilisation en vue d'être stérilisé à l'oxyde d'éthylène.

Le troisième exemple de prothèse implantable (non représentée) comprend une pièce textile tricotée à partir de fils monofilamentaires en polyéthylène téréphtalate de diamètre 0,12 mm qui est stabilisée (ou autrement dit thermofixée) sur rame. La pièce textile tricotée est tricotée sur un métier à tricoter Rachel Karl Meyer comportant 20 aiguilles au pouce (un pouce est égal à 25,4 mm) selon le schéma de mailles représenté à la figure **4****.** La pièce textile tricotée comprend avantageusement uniquement des mailles ouvertes et à une masse surfacique de 60 g/m². Après l'étape de tricotage, le panneau tricoté en sortie du métier à tricoter (ayant éventuellement subit une découpe préliminaire) subit un traitement thermique à 200°C sur une rame textile pendant au moins 60 secondes, puis est refroidi à température ambiante, afin de le stabiliser dans une forme plane. La pièce tricotée est ensuite découpée sur une table, dans le panneau tricoté stabilisé, à l'aide d'un laser selon des dimensions 200 mm x 200 mm.

La pièce textile tricotée est ensuite placée sur une table d'enduction afin d'être enduite d'une composition à base de miel(s) à l'aide d'une barre avec fil enroulé de « Mayer » de largeur **L2** de 400 mm comportant un diamètre de fil de 1,00 mm représenté à la figure **5****.** Dans cet exemple précis, la composition est constituée de 98% en masse de miel de trèfle (de masse volumique 1,40 grammes/cm3) et de 2% en masse de gelée royale provenant de l'abeille Apis Mellifera, qui ont été, préalablement à leur utilisation, filtrés puis stérilisés aux rayonnements gamma à une dose de 25 KGy.

Le revêtement déposé forme un film continu et présente une masse surfacique de l'ordre de 100 g/m².

La prothèse implantable comprenant la pièce textile et le revêtement issu du film de la composition qui a été déposée, est ensuite placée dans un double sachet de stérilisation en vue d'être stérilisée à l'oxyde d'éthylène.

## Revendications

1. Prothèse implantable (1,60), notamment pour la chirurgie pariétale, **caractérisée en ce que** ladite prothèse comprend une pièce textile poreuse (3,62) comprenant des fils monofilamentaires et ayant au moins une première face (3a), et au moins un revêtement fonctionnel (26), continu ou discontinu, disposé selon tout ou partie de ladite première face, ledit revêtement est dans une composition comprenant un miel ou un mélange de plusieurs miels (25) représentant au moins 50% en masse de la masse totale de ladite composition, et **en ce que** la masse surfacique de la pièce textile (3,62) sans revêtement (26) est inférieure ou égale à 150 g/m².

2. Prothèse implantable (1,60) selon la revendication 1, **caractérisée en ce que** ledit miel ou le mélange de plusieurs miels représente au moins 80% en masse de la masse totale de ladite composition (25).

3. Prothèse implantable (1,60) selon l'une ou l'autre des revendications 1 et 2, **caractérisée en ce que** ladite composition (25) comprend au moins 0,5 µg (micro gramme) de defensin-1 pour un gramme dudit au moins un miel ou dudit au moins un mélange de plusieurs miels.

4. Prothèse implantable (1,60) selon l'une quelconque des revendications 1 à 3, **caractérisée en ce que** ladite composition (25) comprend au plus 100 mg (milligramme) de méthylglyoxal pour un kilogramme dudit au moins un miel ou dudit au moins un mélange de plusieurs miels.

5. Prothèse implantable (1,60) selon l'une quelconque des revendications 1 à 4, **caractérisée** en ce ledit revêtement (25) représente au moins 50% en masse de la masse totale de la pièce textile (3,62) revêtue dudit revêtement (25).

6. Prothèse implantable (1,60) selon l'une quelconque des revendications 1 à 5, **caractérisée en ce que** ledit miel ou ledit mélange de plusieurs miels est/sont choisis dans la liste comprenant : le miel monofloral de trèfle, le miel monofloral de vipérine, le miel monofloral d'acacias ou leur mélange, en particulier le miel de manuka est exclu de ladite liste.

7. Prothèse implantable (1,60) selon l'une quelconque des revendications 1 à 6, **caractérisée en ce que** ladite composition comprend de la gelée royale, en particulier d'Apis Mellifera.

8. Prothèse implantable (1,60) selon l'une quelconque des revendications 1 à 7, **caractérisée en ce que** ladite composition (25) comprend au moins un polymère biocompatible liquide à une température supérieure ou égale à 10°C et à une température inférieure ou égale à 70°C, notamment le polycaprolactone et/ou le polymère d'acide lactique de forme D (PDLA).

9. Prothèse implantable (1,60) selon la revendication 8, **caractérisée en ce que** ledit au moins un polymère biocompatible est résorbable.

10. Prothèse implantable (1,60) selon la revendication 1, **caractérisée en ce que** ladite pièce textile (3,62) sans revêtement (26) a une masse surfacique inférieure ou égale à 80 g/m², de préférence inférieure ou égale à 50 g/m², encore de préférence supérieure ou égale à 30 g/m².

11. Prothèse implantable (1,60) selon l'une quelconque des revendications 1 à 10, **caractérisée en ce que** la pièce textile est une pièce tricotée comprenant des mailles ouvertes et des mailles fermées et **en ce que** la proportion en nombre de mailles ouvertes par rapport au nombre total de mailles ouvertes et de mailles fermées pour une pièce textile (3,62) de 100 cm² (10 cm * 10 cm) est supérieure ou égale à 50%, encore de préférence supérieure ou égale à 80%.

12. Procédé de fabrication d'une prothèse implantable (1,60) selon l'une quelconque des revendications 1 à 11, **caractérisée en ce qu'**il comprend les étapes suivantes :
(i) fourniture d'une pièce textile poreuse (3,62) ayant au moins une première face (3a), et d'une composition biocompatible comprenant un miel ou un mélange de miels (25) ;
(ii) application de ladite composition (25) à l'état liquide, éventuellement préalablement chauffée à une température supérieure ou égale à 25°C, sur au moins ladite première face (3a) en sorte de former un revêtement fonctionnel (26), continu ou discontinu;
(iii) optionnellement refroidissement de ladite pièce textile (3,62) revêtue;
(iv) stérilisation de ladite pièce textile (3,62) revêtue.

13. Procédé selon la revendication 12, **caractérisé en ce que** la pièce textile (3,62) est plane, et comprend des première (3a) et seconde (3b) faces opposées, et **en ce que** ladite composition (25) est appliquée sous forme d'un film mince (26) selon la première face (3a) à l'aide d'une filière d'extrusion (28) comprenant au moins une fente longitudinale d'extrusion (30) de ladite composition (25), en particulier de forme rectangulaire, ayant un axe longitudinal L, ladite filière (28) se déplaçant au-dessus de la pièce textile (3) selon une direction T transversale à l'axe longitudinal L.

14. Procédé de fabrication selon l'une quelconque des revendications 12 et 13, **caractérisé en ce que** le miel ou le mélange de plusieurs miels a subit une étape de filtration pour ôter les impuretés solides et une étape de stérilisation avant l'étape (ii), notamment par exposition à des rayons gamma.
